# EUROPEAN PATENT SPECIFICATION

(11) **EP 2 734 557 B1**
(45) Date of publication and mention of the grant of the patent: **07.09.2022**
(21) Application number: 12814463.1
(22) Date of filing: 19.07.2012
(51) Int. Cl.: C08F 251/00, A61K 9/00, A61K 9/06, A61K 47/10, A61K 31/167

(54) **PRODUCTION OF THERMOREVERSIBLE HYDROGELS FOR THERAPEUTIC APPLICATIONS**
HERSTELLUNG THERMOREVERSIBLER HYDROGELE FÜR THERAPEUTISCHE ANWENDUNGEN
PRODUCTION D'HYDROGELS THERMORÉVERSIBLES POUR DES APPLICATIONS THÉRAPEUTIQUES

(30) Priority: 20.07.2011 US 201161509637 P
(43) Date of publication of application: 28.05.2014
(73) Proprietor: UroGen Pharma Ltd., Ra'anana 4365007 (IL)
(72) Inventor: DE LA ZERDA, Jaime, 34751 Haifa (IL); DOLLBERG, Yosh, 43362 Raanana (IL); SHPOLANSKY, Uri, Karkur, 37038 (IL); MALCHI, Nadav, 43216 Ra'anana (IL); HAKIM, Gil, 43596 Ra'anana. (IL); KONORTY, Marina, 46446 Hertzlya (IL)
(74) Representative: Pohlman, Sandra M.
(86) International application number: PCT/IL2012/000283
(87) International publication number: WO 2013/011503

(56) References cited:
- EP-A1- 0 551 626
- WO-A1-2005/110466
- WO-A2-00/07603
- WO-A2-2011/049958
- US-A1- 2010 022 661
- US-A1- 2010 055 153
- US-A1- 2011 008 456
- US-B2- 6 863 859
- SHARMA P K ET AL: "Effect of pharmaceuticals on thermoreversible gelation of PEO-PPO-PEO copolymers", COLLOIDS AND SURFACES. B, BIOINTERFACES, ELSEVIER, AMSTERDAM, NL, vol. 63, no. 2, 1 June 2008 (2008-06-01), pages 229-235, XP022613816, ISSN: 0927-7765, DOI: 10.1016/J.COLSURFB.2007.12.009 [retrieved on 2007-12-23]
- ESCOBAR-CHAVEZ J : ET AL: "Applications of thermo-reversible pluronic F-127 Gels in Pharmaceutical Formulations", JOURNAL OF PHARMACY & PHARMACEUTICAL SCIENCES, CANADIAN SOCIETY FOR PHARMACEUTICAL SCIENCES, CA, vol. 9, no. 3, 1 January 2006 (2006-01-01) , pages 339-358, XP002415851,
- GIULIA BONACUCINA,MARCO CESPI, GIOVANNA MENCARELLI, GIANFABIO GIORGIONI,GIOVANNI FILIPPO PALMIERI: "Thermosensitive Self-Assembling Block Copolymers as Drug Delivery Systems", POLYMERS, 19 April 2011 (2011-04-19), pages 779-811, XP002742516, DOI: 10.3390/polym3020779
- Anonymous: "Nanotechnology in Drug Delivery", 2009, Springer * page 547 *
- M Muthukumaran ET AL: "A REVIEW ON POLYMERS USED IN MUCOADHESIVE DRUG DELIVERY SYSTEM", Int. J. Pharm & Ind, vol. 1, no. 2, 1 June 2011 (2011-06-01), pages 122-127, XP055535284,

## Description

### FIELD OF THE INVENTION

The present invention relates in general to methods for producing sterile thermoreversible hydrogels for therapeutic applications. It relates in particular to a method for producing a sterile thermoreversible hydrogel at a temperature near that at which the viscosity of the hydrogel is at its minimum.

### BACKGROUND OF THE INVENTION

Aqueous liquids that can be applied at room temperature in a free flowing state but that form a semi-solid gel when warmed to body temperature have been reported in the literature for various therapeutic applications, including drug delivery, cell encapsulation and tissue repair. Such thermoreversible systems can be introduced into the body in a minimally invasive manner prior to their solidifying within the desired tissue, organ or body cavity, and provide greater retention at the site of application than the use of a free flowing vehicle.

Among thermoreversible systems, polysaccharides (e.g., cellulose derivatives, xyloglycan, chitosan), N-isopropylacrylamide and poloxamers have commonly been described in the literature. Poloxamers were introduced in the late 1950s and since then they have been proposed for use in diverse pharmaceutical applications. Aqueous poloxamer gels comprise at least water and poloxamer. Many other ingredients are commonly added to the gels including pharmaceutically active ingredients, cosmetic ingredients, surfactants, humectants, moisturizers, emollients, preservatives, antioxidants, buffers, rheology modifiers, colorants, and fragrances.

Two manufacturing processes for aqueous poloxamer gels are known in the art (see, for example, BASF Technical Briefs "Technical Information on Pluronic^{®}" and "Technical Information on Lutrol^{®}"). One method is known as the "hot process" in which water and poloxamer are mixed and then heated to about 80 °C allowing the poloxamer to melt into the hot water. The second method is known as the "cold process" in which water and poloxamer are mixed in cold water, e.g. from 5 °C - 10 °C. Mixing continues at that temperature until the poloxamer is completely dissolved.

For most therapeutic applications, the process of manufacture of thermoreversible hydrogels is required to result in a sterile product. Practical challenges of manufacture may impact the cost and safety of the product, however. This is particularly the case in the manufacture of sterile thermoreversible hydrogels. Their relatively high viscosity and their tendency to solidify following temperature elevation make cost effective manufacture particularly challenging.

WO 00/07603 A2 discloses a pharmaceutic composition includes a pharmaceutically acceptable carrier, comprising a reverse thermally viscosifying polymer.

There thus remains a long-felt need for a simple and cost-effective method for producing sterile thermoreversible hydrogels.

### SUMMARY OF THE INVENTION

The invention herein disclosed is designed to meet this need. In the method herein disclosed, a thermoreversible hydrogel is produced under aseptic conditions at a temperature near that at which its viscosity is at its minimum. The present invention is defined by the independent claims.

It is therefore an object of the present invention to disclose a method for production of a sterile thermoreversible hydrogel, said hydrogel having a temperature Tₘᵢₙ, lower than its gelation temperature, at which the viscosity reaches an at least local minimum, wherein said process comprises: dissolving in water, within a predetermined temperature range relative to said Tₘᵢₙ, components of said hydrogel; mixing said components until a thermoreversible gel is obtained; and filtering said thermoreversible gel within a predetermined temperature of said Tₘᵢₙ.

It is a further object of this invention to disclose a method for production of a sterile thermoreversible hydrogel, said hydrogel having a temperature Tₘᵢₙ at which the viscosity reaches an at least local minimum, wherein said process comprises: dissolving components of said sterile thermoreversible hydrogel in a quantity of water sufficiently large to prevent formation of a gel with thermal gelation characteristics; filtering said gel; and concentrating said gel by evaporating at least part of said water under vacuum, thereby forming a thermoreversible gel.

In one embodiment, said components comprise: between 20% and 30% (w/w) ethylene oxide/propylene oxide block copolymer; between 0.05% and 0.5% (w/w) hydroxypropylmethylcellulose (HPMC); between 0.1% and 2.5% (w/w) PEG-400; and, the balance water.

According to the present invention, said components comprise between 18% and 40% (w/w) ethylene oxide/propylene oxide block copolymer; between 0.05% and 2% (w/w) HPMC; between 0.1% and 10% (w/w) PEG-400; and the balance water.

According to the present invention, said components comprise between 18% and 40% (w/w) ethylene oxide/propylene oxide block copolymer; between 0.05% and 2% (w/w) carboxymethylcellulose sodium (CMC); between 0.1% and 10% (w/w) PEG-400; and the balance water.

According to the present invention, said predetermined temperature range is ± 4 °C of said Tₘᵢₙ. In some embodiments of the invention, said predetermined temperature range is ± 3 °C of said Tₘᵢₙ. In some embodiments of the invention, said predetermined temperature range is ± 2 °C of said Tₘᵢₙ. In some embodiments of the invention, said predetermined temperature range is ± 1 °C of said Tₘᵢₙ. In some embodiments of the invention, said predetermined temperature range is between T_{low} and Tₘᵢₙ inclusive, T_{low} < Tₘᵢₙ.

It is a further object of this invention to disclose such a method as defined in any of the above, further comprising a step of concentrating said thermoreversible gel by evaporation of at least part of said water.

It is a further object of this invention to disclose such a method as defined in any of the above, wherein the steps of said method are performed in a clean room meeting or exceeding the FED-STD-209E requirements for a class 100000 clean room.

It is a further object of this invention to disclose such a method as defined in any of the above, wherein the steps of said method are performed using depyrogenized and/or sterilized utensils.

It is a further object of this invention to disclose such a method as defined in any of the above, further comprising a step of flowing said gel to a filter, said step of flowing occurring between said step of processing and said step of filtering.

It is a further object of this invention to disclose such a method as defined in any of the above, wherein said step of filtering is performed aseptically.

It is a further object of this invention to disclose such a method as defined in any of the above, further comprising a step of sterilizing said thermoreversible hydrogel in an autoclave after said step of filtering.

It is a further object of this invention to disclose such a method as defined in any of the above, further comprising a step of sterilizing said thermoreversible hydrogel by irradiation after said step of filtering.

It is a further object of this invention to disclose such a method as defined in any of the above, further comprising a step of sterilizing said thermoreversible hydrogel by irradiation after said step of filtering.

According to the present invention, said components comprise a reverse thermal gelation agent.

According to the present invention, the reverse thermal gelation agent comprises ethylene oxide/propylene oxide block copolymer.

It is a further object of this invention to disclose such a method as defined in any of the above, wherein said components additionally comprise at least one component chosen from the group consisting of adhesive and thickening compounds; bonding agents; pH-modifying substances; diffusion coatings; plasticizers; water soluble polymers; water-soluble substances chosen from the group consisting of urea, salts, sugars, sugar alcohols, and any combination thereof; swellable excipients; matrix forming polymers; tight junction modifiers; permeability enhancers; surfactants; charged polymers; poly(propylene oxide) (PPO), poly(lactide-co-glycolic acid) (PLGA), poly(N-isopropylacrylamide) (PNIPAM), poly(propylene fumarate) (PPF), polyurethane (PU), poly(organophosphazene) (POP), stearic acid, poly(acrylic acid), glyceryl stearate, cetearyl alcohol, sodium stearoyl lactylate, hydroxy-lanolin, dimethyl sulfoxide; decylmethyl sulfoxide; tert-butylcyclohexanol; fatty acids; fatty acid esters; fatty acid salts; ethanol; nicotinamide; perfluoropolyethers; monoterpene ketones; and tris(hydroxymethyl)aminomethane.

In some embodiments of the invention, said bonding agent is selected from the group consisting of polycarbophil, cellulose, microcrystalline cellulose, cellulose derivatives, dicalcium phosphate, lactose, sucrose ethylcellulose, hydroxypropymethylcellulose acetate succinate (HPMCAS), PVP, vinylpyrrolidone/vinyl acetate copolymer, polyethylene glycol, polyethylene oxide, polymethacrylates, polyvinyl alcohols (PVA), partially hydrolysed polyvinyl acetate (PVAc), polysaccharides, fats and fatty acid derivatives and any combination thereof.

In some embodiments of the invention, said pH-modifying substance is chosen from the group consisting of adipic acid, malic acid, L-arginine, ascorbic acid, aspartic acid, benzenesulfonic acid, benzoic acid, succinic acid, citric acid, ethanesulphonic acid, 2-hydroxyethanesulphonic acid, fumaric acid, gluconic acid, glucuronic acid, glutamic acid, potassium hydrogen tartrate, maleic acid, malonic acid, methanesulfonic acid, toluenesulfonic acid, trometamol, tartaric acid, hydrochloric acid, sodium hydroxide, phosphate salts, tris and any combination thereof.

In some embodiments of the invention, said diffusion coating is selected from the group consisting of ethylcelluloses and polymethacrylates such as, for example, EUDRAGIT NE, EUDRAGIT RS and RL, cellulose acetate and cellulose acetate butyrate or any combination thereof.

In some embodiments of the invention, said plasticizer is selected from the group consisting of citric acid derivatives, phthalic acid derivatives, benzoic acid, benzoic esters, other aromatic carboxylic esters, trimellithic esters, aliphatic dicarboxylic esters, dialkyl adipates, sebacic esters, tartaric esters, glycerol monoacetate, glycerol diacetate, glycerol triacetate, polyols, fatty acids and derivatives thereof, glycerol monostearates, acetylated fatty acid glycerides, natural oils, miglyol, fatty acid alcohols, cetyl alcohol, cetylstearyl alcohol, and any combination thereof.

In some embodiments of the invention, said water-soluble substance comprises at least one salt chosen from the group consisting of sodium chloride, potassium chloride, and ammonium chloride.

In some embodiments of the invention, said water-soluble substance comprises at least one sugar chosen from the group consisting of sucrose, lactose, glucose, fructose, and maltose. In some embodiments of the invention, said water-soluble substance comprises at least one sugar alcohol chosen from the group consisting of mannitol, sorbitol, xylitol, and lactitol.

In some embodiments of the invention, said water-soluble polymer is selected from the group consisting of polyethylene glycols, PVP, PVA, HPC, hydroxyethylcelluloses (HEC), MC, dextrins, maltodextrins, cylcodextrins, dextrans, and any combination thereof.

In some embodiments of the invention, said swellable excipient is chosen from the group consisting of polyvinylpyrrolidones, crospovidones, crosslinked sodium carboxymethylcellulose, crosslinked sodium carboxymethylstarch, polyethylene oxides, polymethyacrylates, low-substituted hydroxypropylmethylcellulose (L-HPC), cellulose acetate, ethylcellulose and polymethacrylates, high-molecular weight polyethylene oxides, xanthan gum, copolymers of vinylpyrrolidone and vinyl acetate, polyvinylpyrrolidones, crospovidones, poly(hydroxyalkyl methacrylate), alginates, galactomannans, and any combination thereof.

In some embodiments of the invention, said matrix forming polymer is selected from the group consisting of hydroxyethylmethylcelluloses, hydroxypropylcelluloses, hydroxyethylcelluloses, methylcelluloses, ethylcelluloses, alkylcelluloses, hydroxyalkyl-celluloses, hydroxyalkylmethylcelluloses, sodium carboxymethylcelluloses, alginates, galactomannans, xanthans, polyethylene oxides, polyacrylic acids, polymethacrylic acids, polymethacrylic acid derivatives, polyvinyl alcohols, partially hydrolysed polyvinyl acetate, polyvinylpyrrolidone, agar, pectin, gum arabic, tragacanth, gelatin, starch, starch derivatives, poly(propylene oxide) (PPO), poly(lactide-co-glycolic acid) (PLGA), poly(N-isopropylacrylamide) (PNIPAM), poly(propylene fumarate) (PPF), polyurethane (PU), poly(organophosphazene) (POP), stearic acid, poly(acrylic acid), glyceryl stearate, cetearyl alcohol, sodium stearoyl lactylate, hydroxy-lanolin, and any combination thereof.

In some embodiments of the invention, said surfactant is chosen from the group consisting of polysorbates, sodium dodecyl sulfate, and dextran sulfate.

In some embodiments of the invention, said charged polymer is chosen from the group consisting of chitosan, polyarginine, polylysine, and alginate.

In some embodiments of the invention, wherein said monoterpene ketone is chosen from the group consisting of (-)menthol, (-)menthone, peppermint oil, and spearmint oil.

It is a further object of this invention to disclose such a method as defined in any of the above, further comprising a step of mixing dry components of said hydrogel in a separate container prior to said step of dissolving components of said hydrogel in water.

It is a further object of this invention to disclose such a method as defined in any of the above, further including a step of adding an effective amount of a therapeutic agent.

### BRIEF DESCRIPTION OF THE FIGURES

The invention disclosed herein is described with reference to the figures, in which:
FIG. 1 presents schematic illustrations of micellar phases formed by the polymer herein disclosed;
FIG. 2 presents the results of measurements of viscosity as a function of temperature for a reverse thermal hydrogel comprising 27% poloxamer PF-127, 0.2% HPMC, and 1% PEG 400 in water for irrigation (WFI);
FIG. 3 presents the results of measurements of viscosity as a function of temperature for a reverse thermal hydrogel comprising 27% poloxamer PF-127 in WFI (triangles) and PF-127 + 0.4% HPMC in WFI (diamonds);
FIG. 4 presents the results of measurements of viscosity as a function of temperature (on a semilogarithmic scale) for two different reverse thermal hydrogels, one comprising 27% poloxamer PF-127, 0.2% HPMC, 1% PEG 400 in water for irrigation (diamonds), and one comprising 20% poloxamer PF-127, 0.2% CMC, 1% PEG 400 (circles);
FIG. 5 presents the same results as are shown in FIG. 4, but with the viscosity plotted on a linear scale.

### DETAILED DESCRIPTION OF THE PREFERRED EMBODIMENTS

In the following description, various aspects of the invention will be described. For the purposes of explanation, specific details are set forth in order to provide a thorough understanding of the invention. It will be apparent to one skilled in the art that there are other embodiments of the invention that differ in details without affecting the essential nature thereof. Therefore the invention is not limited by that which is illustrated in the figure and described in the specification, but only as indicated in the accompanying claims, with the proper scope determined only by the broadest interpretation of said claims.

The current invention is directed to a process for efficient manufacture of sterile thermoreversible hydrogels. The process comprises of solubilization/dissolution of the thermoreversible hydrogel ingredients and filtration of the hydrogels.

The thermoreversible hydrogel viscosity is strongly dependent on the hydrogel temperature. Measurement of the dependence of viscosity on temperature is critical for the manufacturing of the hydrogel formulation especially for large-scale manufacturing and mass production. Determination of the hydrogel viscosity as a function of temperature provides the formulation gelation temperature, which serves as the practical upper limit of the gel manufacturing temperature with a sufficient safety margin to ensure that the gel will not solidify while being processed (for example, during the filling or filtration steps). In addition, depending on the gel formulation, the hydrogel viscosity - temperature profile may reveal a minimum viscosity point that can be exploited at various manufacturing steps in order, for example, to enable filtration of the formulation and to minimize the time required for manufacturing, energy requirements, and manufacturing costs. This minimum viscosity point at Tₘᵢₙ can be easily overlooked when the viscosity is plotted as a function temperature in a semilogarithmic scale (as usually done), yet will be apparent when the viscosity is plotted as a function of temperature in a linear scale.

As used herein, the term "viscosity minimum" of a reverse thermal gelation formulation refers to a minimum in the viscosity as a function of temperature, occurring at temperature Tₘᵢₙ, at which temperature the viscosity is at least 5% lower than the maximum viscosity over the temperature range of ± 4 °C relative to Tₘᵢₙ. By this definition, while the term can refer to a global minimum in the viscosity, a local minimum in the viscosity as a function of temperature is not excluded from the meaning of the term as used definition.

In a preferred embodiment of the present invention the solubilization/dissolution of the thermoreversible hydrogel ingredients is performed in a temperature control mixer at a temperature range around Tₘᵢₙ in which the hydrogel viscosity is minimal. This procedure is performed in a clean room (class 100,000 room) using depyrogenized and sterile utensils to ensure minimal microbial load of the gel and enable sterilization. In preferred embodiments of the invention, in order to minimize the time needed to dissolve the components of the gel, the solid ingredients are mixed in a separate container and then added to the water portion that was precooled to at a temperature range around Tₘᵢₙ. Following the dissolution of the components of the gel and mixing until a homogeneous gel is obtained, the gel is aseptically filtered and filled in vials. The filtration does not necessarily occur at the same physical location, and the gel can be brought from the container in which it is formed to the filtration apparatus by any method known in the art, e.g. by using a peristaltic pump to pump the gel through flexible tubing to the filter.

As discussed previously, for the majority of therapeutic uses, government regulations require that the hydrogel product be sterile. Thus, the method herein disclosed is drawn to a novel method of production of a sterile thermoreversible hydrogel. Sterilization of the gel product can be performed using any method known in the art. Typical sterilization methods useful for the present invention include aseptic filtration, autoclaving, and irradiation.

In preferred embodiments of the invention, aseptic filtration is used. In the most preferred embodiments of the invention, the gel is filtered through at least one 0.2 micron membrane in a clean environment (at least class 100). The advantage of aseptic filtration as a final sterilization step is that since relatively much lower energy is applied to the gel during the process (in comparison to autoclaving or irradiation), gel ingredient degradation due to polymer chain breakage that typically occurs following autoclaving or radiation sterilization can be avoided. Thus, following the aseptic filtration process, the gel will usually retain its pre-filtration characteristics such as component concentrations, viscosity, and stability. Furthermore, if the gel includes pharmaceutically active ingredients such as drugs, then aseptic filtration will be the sterilization method of choice in order to minimize the degradation of the active ingredients.

The filtration procedure of a thermoreversible hydrogel is technically challenging due to relatively high viscosity of such gels, and to their tendency to solidify as the temperature is raised. Gel heating leading to solidification can result from the shearing stress of the gel by the tubing walls through which the gel flows, and especially due to its shearing through the small membrane pores of the filter. The tubes and filter can be clogged by the viscous or solid gel making the streaming and filtration of the gel unfeasible.

Thus, in preferred embodiments of the invention, the aseptic filtration is performed in a temperature controlled environment in which the tubing temperature as well as the filter temperature is set to a temperature below the gelation temperature with sufficient safety margins. In some embodiments of the invention disclosed herein, at least one of the tubing and the filter are maintained to within a predetermined temperature range around Tₘᵢₙ. In preferred embodiments of the invention, at least one of the tubing and the filter are maintained to within ± 4 °C of Tₘᵢₙ. In yet more preferred embodiments of the invention, at least one of the tubing and the filter are maintained to within ± 3 °C of Tₘᵢₙ. In even more preferred embodiments of the invention, at least one of the tubing and the filter are maintained to within ± 2 °C of Tₘᵢₙ. In yet more preferred embodiments of the invention, at least one of the tubing and the filter are maintained to within ± 1 °C of Tₘᵢₙ. Streaming the gel and filtration of the gel at temperatures near Tₘᵢₙ will enable the filtration of high viscosity gel formulation, increase the gel flux, decrease manufacturing time, and reduce manufacturing costs. In other preferred embodiments of the invention, the tubing and/or filter are maintained at a predetermined temperature T_{low} ≤ Tₘᵢₙ.

According to the present invention, the thermoreversible gel comprises at least one poloxamer. Poloxamers, nonionic triblock copolymers known for their thermal reversible gelation properties, are composed of a central hydrophobic chain of polyoxypropylene (poly(propylene oxide)) flanked by two hydrophilic chains of polyoxyethylene (poly(ethylene oxide)) represented by the following chemical structure: 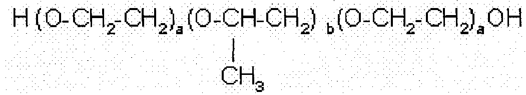 (a) and (b) represent the number of repeating units of ethylene oxide and propylene oxide, respectively. Values of (a) and (b) in typical commerically available poloxamers are given in Table 1.

**Table 1**

| Pluronic/Lutrol | Poloxamer | (a) | (b) |
|---|---|---|---|
| L44 | 124 | 12 | 20 |
| F68 | 188 | 80 | 27 |
| F87 | 237 | 64 | 37 |
| F108 | 338 | 141 | 44 |
| F127 | 407 | 101 | 56 |

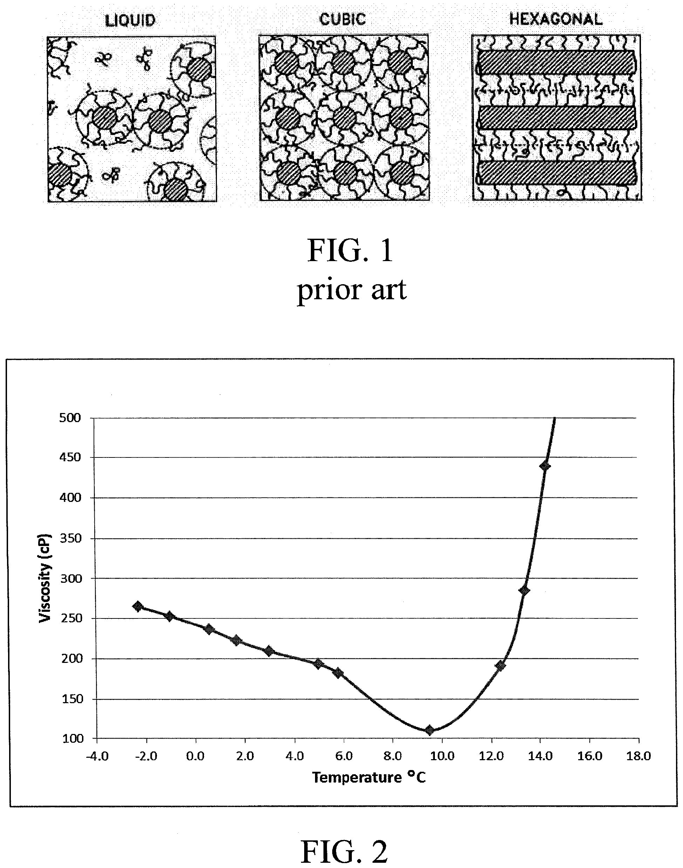

Poloxamer 407 (PF-127) is recognized as a substance (Pharmacopeia/NF) suitable for pharmaceutical use. It has a good solubilizing capacity and low toxicity and is therefore considered a good medium for drug delivery systems. The "F" designation refers to the flake form of the product. Pluronic 127 is commercially available as LUTROL^{®} F127 (BASF laboratories, Wyandoote, USA) and SYNPERONIC^{®} F127 (ICI laboratories, Wilton, UK), and has a molecular weight of about 12,600 (9,840-14,600); (a) and (b) are equal to 95-105 and 54 - 60, respectively. PF-127 is more soluble in cold water than in hot water as a result of increased salvation and hydrogen bonding at lower temperatures. PF-127 aqueous solutions of 20 to 30% w/w have the characteristic of reverse thermal gelation, i.e., they are liquid at low temperatures (4-5 °C), but gel upon warming to room temperature. The gelation is reversible upon cooling. At low temperatures in aqueous solutions, a hydration layer surrounds PF-127 molecules. When the temperature is raised, the hydrophilic chains of the copolymer become desolvated as a result of the breakage of the hydrogen bonds that had been formed between the solvent and these chains. This phenomenon favors hydrophobic interactions among the polyoxypropylene domains and leads to gel formation. Because of the dehydration process, the hydroxyl groups become more accessible. A liquid micellar phase is stable at low temperatures but transforms into a cubic structure with increasing temperature. At higher temperatures, a phase of hexagonal packed cylinders is formed. Reference is now made to FIG. 1, which illustrates these three structures.

When the thermoreversible hydrogel formulation includes poloxamer, the solubilization/dissolution of the poloxamer can be performed using either the hot dissolution process or the cold dissolution process. In typical embodiments in which the cold dissolution process is used, the poloxamer is dissolved at a temperature of about 5 °C to about 10 °C in water. In typical embodiments in which the hot dissolution process is used, the polymer is added to water heated to a temperature of about 75 °C to about 85 °C with slow stirring until a clear homogeneous solution is obtained.

In some embodiments of the invention herein disclosed, the reverse thermal hydrogel also includes an effective amount of a therapeutic agent.

In some embodiments of the invention, the reverse thermal gelation formulation including poloxamer contains at least one additional component, such as adhesive and thickening compounds; bonding agents; pH-modifying substances; diffusion coatings; plasticizers; matrix permeability increasing components; swellable excipients; matrix-forming polymers; diffusion-controlled or pulsatile formulations; reverse thermal gelation agents; permeability enhancers, or any combination thereof. In general, these additional components may be added at any stage of the process.

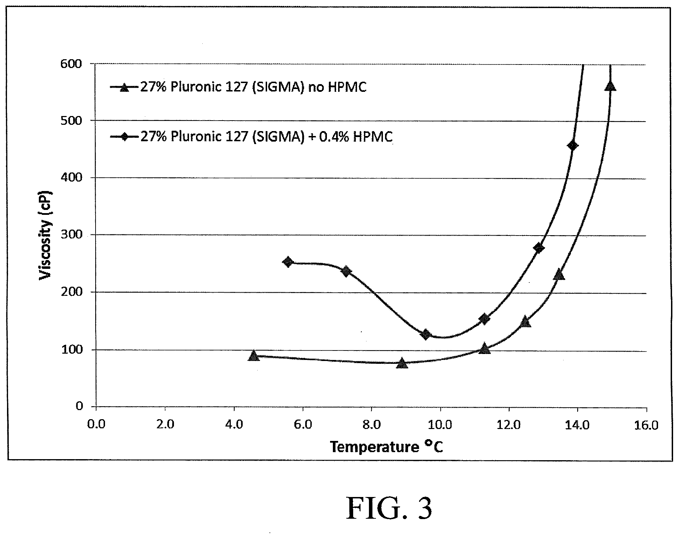

Reference is now made to FIG. 2, which presents results of viscosity measurements as a function of temperature for an exemplary embodiment of a thermoreversible hydrogel formulation comprising 27% (w/w) poloxamer, 0.2% (w/w) hydroxypropylmethylcellulose (HPMC) and 1% (w/w) polyethylene glycol (PEG-400). The plot demonstrates a minimum in the gel viscosity at Tₘᵢₙ = 9.5 °C. Tₘᵢₙ was found to be dependent of the HPMC concentration. Reference is now made to FIG. 3, which presents results of viscosity measurements as a function of temperature for a hydrogel formulation that does not include HPMC, but only Pluronic F127. As can be seen from the graph, for this formulation, no viscosity minimum can be detected.

Addition of HPMC to the hydrogel results in an overall increase of the gel viscosity at all temperatures, and a viscosity minimum at Tₘᵢₙ = 9 °C - 10 °C. In the particular example shown in FIG. 3, Tₘᵢₙ = 9.5 °C. The viscosity at Tₘᵢₙ is 43% lower than that at 4.7 °C (110 mPa s vs. 193 mPa s) and 58% lower than the viscosity at -2.3 °C (265 mPa s vs. 110 mPa s). Thus, manufacture of the hydrogel at a temperature near Tₘᵢₙ will accelerate the dissolution of the formulation, lower the energy required to make the gel flow, and, significantly for production of a sterile hydrogel, it will enable aseptic filtration of the formulation. Alternatively, the manufacturing process can be performed at a temperature below Tₘᵢₙ at which the viscosity is still low enough to enable aseptic filtration of the gel.

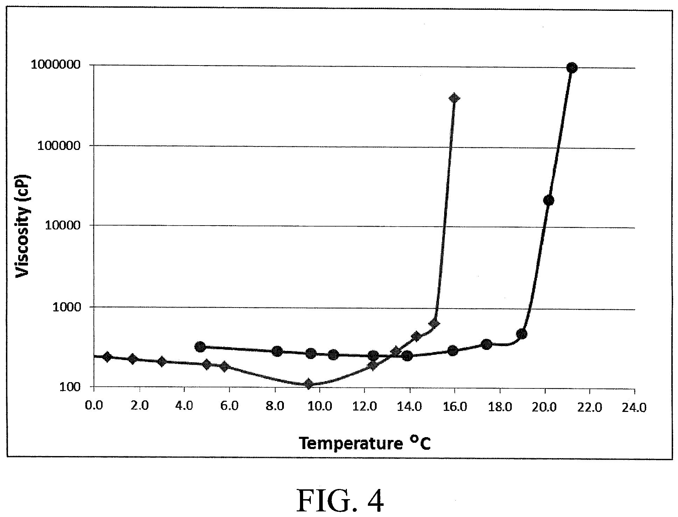

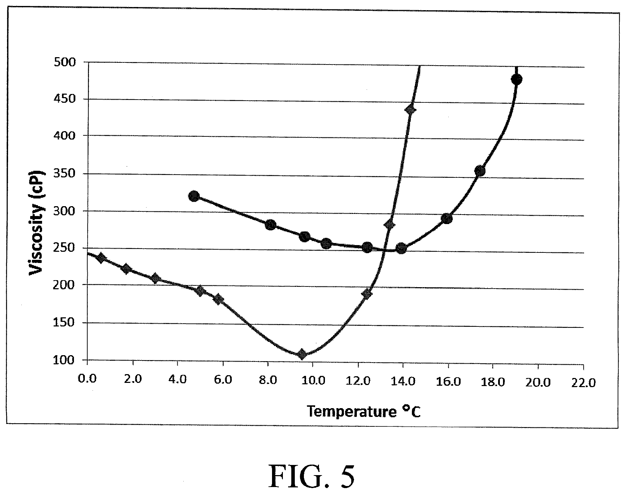

As an additional non-limiting example of an embodiment of the invention herein disclosed, reference is now made to FIG. 4, which presents a semi-logarithmic plot of the viscosity as a function of temperature for a reverse thermal gelation hydrogel comprising 20% (w/w) PF-127 poloxamer, 0.2% (w/w) carboxymethyl cellulose sodium (CMC-sodium) and 1% PEG-400 (circles) and FIG. 5 which presents the same data on a linear scale. As a comparison, results for a Pluronic-HMPC-PEG formulation are plotted on the two graphs as diamonds. Such curves are normally presented on semilogarithmic plots, but, as can be seen in FIG. 4, the presence of a minimum in the gel viscosity at Tₘᵢₙ can be easily overlooked on such a plot. In contrast, when the results are plotted on a linear scale, as shown in FIG. 5, a clear minimum viscosity point can be observed also for the Pluronic-CMC-PEG formulation at Tₘᵢₙ = 12.4 °C - 13.9 °C. Thus, in a preferred embodiment of the method disclosed herein, the manufacture of this formulation is performed at 12.4 °C, where the viscosity is 21% lower than the viscosity at 4.7 °C (254 mPa s vs.321 mPa s), in order to accelerate the dissolution of the formulation, lower the energy required to make the gel flow, and enable sterilization of the formulation by aseptic filtration.

In another preferred embodiment of the invention herein disclosed, the method of producing a thermoreversible hydrogel comprises producing the hydrogel in a temperature-controlled mixer operating in a predetermined temperature range around Tₘᵢₙ. In a more preferred embodiment, the temperature range is ± 4 °C relative to Tₘᵢₙ. In an even more preferred embodiment, the temperature range is ± 3 °C relative to Tₘᵢₙ. In a still more preferred embodiment, the temperature range is ± 2 °C relative to Tₘᵢₙ. In a most preferred embodiment, the temperature range is ± 1 °C relative to Tₘᵢₙ. This procedure is performed in a clean room (class 100,000 room) using depyrogenized and sterile utensils to ensure minimal microbial load of the gel and enable final sterilization. A typical (non-limiting) example of a thermoreversible hyrogel that can be produced in this manner comprises poloxamer and CMC and/or HPMC. To minimize dissolution time, the solid ingredients are mixed and then added to the water that has been precooled to within a predetermined temperature of Tₘᵢₙ. Following the dissolution of the ingredients and formation of a homogeneous gel, the gel is aseptically filtered and transferred to containers (e.g. vials) for storage. As described above, the gel may be brought to the filtration apparatus by any means known in the art. In preferred embodiments, a peristaltic pump is used to pump the gel through flexible tubing from the container in which it is formed to the filter. In some embodiments of the invention, the method further includes addition of an effective amount of a therapeutic agent.

In an alternative embodiment of the invention, the method comprises preparation of a dilute solution of the gel product with a much lower viscosity, performing the aseptic filtration at optimal viscosity conditions (in particular, a temperature near Tmin), and then concentrating the mixture to the desired level by evaporation of the excess water under vacuum. In a preferred embodiment of this invention the formulation is diluted sufficiently (the typical Pluronic F127 in this embodiment is <18% w/w) such that it ceases to exhibit reverse thermal gelation, and will thus fail to solidify at temperatures above Tₘᵢₙ. Only with the final concentration step will the hydrogel become thermoreversible. This embodiment of the method is particularly useful for cases in which, due to the particular equipment used or the viscosity of the particular hydrogel formulation being produced, the filtration procedure does not provide a sufficiently high flux to be useful, or if the filtration equipment clogs despite the optimal filtration conditions used (Tₘᵢₙ).

In some embodiments of the invention herein disclosed, the method further comprises a step of adding reverse thermal gelation agents. In preferred embodiments of the invention, the reverse thermal gelation compositions are selected from a group consisting of Poloxamers, methylcellulose, hydroxypropylmethylcellulose, or any combination thereof, Aliginates, pH controlled gelation agent as cellulose acetophathalate and carbopol, and any combination thereof. In preferred embodiments of the invention in which the reverse thermal gelation agents are poloxamers, the polaxmers are chosen from the group consisting of Poloxamer 407, Poloxamer 188, Poloxamer 338, and any combination thereof.

In yet another embodiment of the invention herein disclosed, the final sterilization of the hydrogel is performed by autoclaving or radiation rather than by aseptic filtration.

In some embodiments of the invention herein disclosed, the method further comprises a step of adding adhesives and/or thickeners. In preferred embodiments of the invention, the adhesive and thickening compounds are selected from the group consisting of polycarbophil, crosslinked poly(acrylic acid), divinyl glycol, hydroxypropylmethylcellulose (HPMC), somepolyvinylpyrrolidone (PVP), methylcellulose (MC), hydroxypropylcellulose (HPC), other hydroxyalkylcelluloses, hydroxyalkylmethylcelluloses, carboxymethylcelluloses and salts thereof, polyacrylic acids, polymethacrylates, gelatin, starch or starch derivatives, gums (e.g. guar gum and xanthan gum), and any combination thereof. In typical embodiments of the invention, these components are added at the mixing stage. If solid adhesives or thickening agents are used, in preferred embodiments of the invention, they are mixed with the other solid components of the gel in a separate container prior to the step of mixing all of the components.

In some embodiments of the invention herein disclosed, the method further comprises a step of adding at least one bonding agent. In preferred embodiments of the invention, the bonding agents are selected from the group consisting of polycarbophil, cellulose, microcrystalline cellulose, cellulose derivatives such as HPMC, HPC and low-substituted hydroxypropylcellulose (L-HPC), dicalcium phosphate, lactose, sucrose, ethylcellulose, hydroxypropymethylcellulose acetate succinate (HPMCAS), PVP, vinylpyrrolidone/vinyl acetate copolymer, polyethylene glycol, polyethylene oxide, polymethacrylates, polyvinyl alcohols (PVA), partially hydrolysed polyvinyl acetate (PVAc), polysaccharides (e.g. alginic acid, alginates, galactomannans) waxes, fats and fatty acid derivatives and any combination thereof. The bonding agents are typically added at any stage of the manufacturing process.

In some embodiments the invention, the method further comprises a step of adding of at least one pH-modifying substance. In preferred embodiments of the invention, the pH-modifying substance is selected from the group consisting of adipic acid, malic acid, L-arginine, ascorbic acid, aspartic acid, benzenesulphonic acid, benzoic acid, succinic acid, citric acid, ethanesulphonic acid, 2-hydroxyethanesulphonic acid, fumaric acid, gluconic acid, glucuronic acid, glutamic acid, potassium hydrogen tartrate, maleic acid, malonic acid, methanesulphonic acid, toluenesulphonic acid, trometamol, tartaric acid, and any combination thereof. In the most preferred embodiments, the pH-modifying substance is chosen from the group consisting of hydrochloric acid, sodium hydroxide, citric acid, succinic acid, tartaric acid, potassium hydrogen tartrate, phosphate salts, tris and any combination thereof. In typical embodiments of the invention in which a pH-modifying agent is used, it is the first ingredient added to the water in the mixing stage of the manufacturing process.

In some embodiments of the invention disclosed herein, the method further comprises a step of providing the thermoreversible hydrogel with a diffusion coating. In preferred embodiments of the invention, the diffusion coating is made of a material selected from the group consisting of ethylcelluloses and polymethacrylates (e.g. EUDRAGIT NE, EUDRAGIT RS, or EUDRAGIT RL), cellulose acetate, cellulose acetate butyrate, and any combination thereof. The diffusion coating may be added at any point in the manufacturing process subsequent to formation of the hydrogel. Any appropriate method known in the art for adding a diffusion coating to a hydrogel can be used.

In some embodiments of the invention, the method further comprises a step of adding at least one plasticizer. In preferred embodiments of the invention, the plasticizer is selected from the group consisting of citric acid derivatives (non-limiting examples include triethyl citrate, tributyl citrate, or acetyl triethyl citrate), phthalic acid derivatives (non-limiting examples include dimethyl phthalate, diethyl phthalate, and dibutyl phthalate), benzoic acid, benzoic esters, other aromatic carboxylic esters, trimellithic esters, aliphatic dicarboxylic esters, dialkyl adipates, sebacic esters (e.g. diethyl sebacate), tartaric esters, glycerol monoacetate, glycerol diacetate, glycerol triacetate, polyols (non-limiting examples include glycerol, 1,2-propanediol, and polyethylene glycol), fatty acids and derivatives thereof, glycerol monostearates, acetylated fatty acid glycerides, natural oils (e.g. castor oil), Miglyol, fatty acid alcohols, cetyl alcohol, cetylstearyl alcohol, and any combination thereof. In typical embodiments of the invention, the plasticizer is added during the mixing stage.

In some embodiments of the invention, the method further comprises a step of adding at least one water-soluble substance. In preferred embodiments of the invention, the water-soluble substance is selected from the group consisting of polyethylene glycols, PVP, PVA, HPMC, HPC, hydroxyethylcelluloses (HEC), MC, carboxymethylcelluloses and their salts, dextrins, maltodextrins, cylcodextrins, dextrans, urea, salts (non-limiting examples include sodium chloride, potassium chloride, and ammonium chloride), sugars (non-limiting examples include sucrose, lactose, glucose, fructose, and maltose), sugar alcohols (non-limiting examples include mannitol, sorbitol, xylitol, and lactitol), and any combination thereof. The water-soluble substance may be added at any stage during the process. In preferred embodiments of the invention that include a step of mixing solid components in a separate container in which the water-soluble substance is a solid, it is mixed with the remaining solid components prior to the step of mixing.

In some embodiments of the invention, the method further comprises a step of adding at least one swellable excipient. In preferred embodiments of the invention, the swellable excipient is selected from the group consisting of polyvinylpyrrolidones, crospovidones, crosslinked sodium carboxymethylcellulose, crosslinked sodium carboxymethylstarch, polyethylene oxides, polymethyacrylates, low-substituted hydroxypropylmethylcellulose (L-HPC), cellulose acetate, ethylcellulose and polymethacrylates, high-molecular weight polyethylene oxides, xanthan gum, copolymers of vinylpyrrolidone and vinyl acetate, polyvinylpyrrolidones, crospovidones, crosslinked sodium carboxymethylcellulose, crosslinked sodium carboxymethylstarch,, poly(hydroxyalkyl methacrylate), alginates and galactomannans and mixtures thereof or any combination thereof. In typical embodiments of the invention, the swellable excipient is mixed in a separate container with the reverse thermal gelation ingredient prior to addition to the water.

In some embodiments of the invention, the method further comprises a step of adding at least one matrix forming polymer. In preferred embodiments of the invention, the matrix forming polymer is selected from the group consisting of is selected from the group consisting of hydroxyethylmethylcelluloses, hydroxypropylcelluloses, hydroxyethylcelluloses, methylcelluloses, ethylcelluloses, alkylcelluloses, hydroxyalkyl-celluloses, hydroxyalkylmethylcelluloses, sodium carboxymethylcelluloses, alginates, galactomannans, xanthans, polyethylene oxides, polyacrylic acids, polymethacrylic acids, polymethacrylic acid derivatives, polyvinyl alcohols, partially hydrolysed polyvinyl acetate, polyvinylpyrrolidone, agar, pectin, gum arabic, tragacanth, gelatin, starch, starch derivatives, poly(propylene oxide) (PPO), poly(lactide-co-glycolic acid) (PLGA), poly(N-isopropylacrylamide) (PNIPAM), poly(propylene fumarate) (PPF), polyurethane (PU), poly(organophosphazene) (POP), stearic acid, poly(acrylic acid), glyceryl stearate, cetearyl alcohol, sodium stearoyl lactylate, hydroxy-lanolin, and any combination thereof. and any combination thereof.

In some embodiments of the invention, the method further comprises a step of adding at least one substance chosen from the group consisting of tight junction modifiers and permeability enhancers as anionic and non-anionic surfectants as Polysorbates, sodium dodecyl sulfate, dextran sulfate, charged polymers as chitosan poly-arginine, polylysine, aliginate, dimethyl sulfoxide (DMSO), decylmethyl sulfoxide, tert-butyl cyclohexanolfatty acids their esters and salts, Ethanol, Nicotinamide or urea, Perfluoropolyether, Monoterpene ketones like (-) menthol, (-) menthone, peppermint oil, spearmint oil, disodium citrate, succinic acid or tris.

The method disclosed in the present invention will now be illustrated by the following non-limiting examples.

### EXAMPLE 1

5 kg of reverse thermal gelation hydrogel was produced according to the following procedure, using the components listed in Table 2.

**Table 2**

| Grade | Raw material | % (w/w) | g/batch |
|---|---|---|---|
| USP | Pluronic F127 or | 27.0 | 1350.0 |
| NF | Poloxamer 407 NF | | |
| USP | Polyethylene glycol 400 (PEG-400) | 1.0 | 50.0 |
| USP | Hydroxypropylmethylcellulose (HPMC) | 0.2 | 10.0 |
| USP | Water for injection (WFI) | 71.8 | 3590.0 |
| Total weight | | 100.0 | 5000.0 |

The hydrogel was produced in a class 100000 clean room using depyrogenized, sterile utensils. A double lumen mixer with a two-wing impeller was precooled to a temperature of 8 ± 2 °C (i.e. near Tₘᵢₙ; see FIG. 2). 3590.0 g of WFI were then transferred into the mixer. The mixer impeller was turned on and 50.0 g of PEG-400 were added to the mixer. 10.0 g of HPMC and 1350.0 g Pluronic F127 were mixed thoroughly in a separate container and then gradually added to the mixer. The gel was stirred for 1 hr until homogeneous, transparent, colorless solution was obtained.

Aseptic filtration was then performed in a Class 100 environment. The mixer outlet was connected to a short sterile silicone tube connected to a peristaltic pump and to a double lumen stainless steel tube connected to a 0.2 micron filter. Both the double lumen tube and the filter were maintained at a temperature of 8 ± 2 °C. The gel was filtered to a receiving double lumen vessel maintained at a temperature of 8 ± 2 °C. The viscosity of the gel following filtration was found to be equal to its viscosity prior to filtration. The filtered gel was then transferred to vials.

### EXAMPLE 2

5 kg of reverse thermal gelation hydrogel was produced according to the following procedure, using the components listed in Table 2.

**Table 2**

| Grade | Raw material | % (w/w) | g/batch |
|---|---|---|---|
| USP | Pluronic F127 or | 20.0 | 1,000.0 |
| NF | Poloxamer 407 NF | | |
| USP | Polyethylene glycol 400 (PEG-400) | 1.0 | 50.0 |
| USP | Carboxymethylcellulose sodium (CMC) | 0.2 | 10.0 |
| USP | Water for injection (WFI) | 78.8 | 3,940.0 |
| Total weight | | 100.0 | 5,000.0 |

The hydrogel was produced in a class 100000 clean room using depyrogenized, sterile utensils. A double lumen mixer with a two-wing impeller was precooled to a temperature of 11 ± 1 °C (i.e. near Tₘᵢₙ; see FIG. 5). 3940.0 g of WFI were then transferred into the mixer. The mixer impeller was turned on and 50.0 g of PEG-400 were and added to the mixer. 10.0 g of CMC and 1000.0 g Pluronic F127 were mixed thoroughly in a separate container and then gradually added to the mixer. The gel was stirred for 1 hr until homogeneous, transparent, colorless solution was obtained.

Aseptic filtration was then performed in a Class 100 environment. The mixer outlet was connected to a short sterile silicone tube connected to a peristaltic pump and to a double lumen stainless steel tube connected to a 0.2 micron filter. Both the double lumen tube and the filter were maintained at a temperature of 11 ± 1 °C. The gel was filtered to a receiving double lumen vessel maintained at a temperature of 11 ± 1 °C. The viscosity of the gel following filtration was found to be equal to its viscosity prior to filtration. The filtered gel was then transferred to vials.

### EXAMPLE 3

A thermoreversible hydrogel was manufactured according to the following procedure, in which a dilute gel formulation with low viscosity was prepared, followed by aseptic filtration and concentration of gel formulation by partial evaporation of water.

The components used to produce this formulation are listed in Table 3.

**Table 3**

| Grade | Raw material | % (w/w) | g/batch (w/w) |
|---|---|---|---|
| USP | Pluronic F127 or | 13.5 | 1350.0 |
| NF | Poloxamer 407 NF | | |
| USP | Polyethylene glycol 400 (PEG-400) | 0.5 | 50.0 |
| USP | Hydroxypropyl methyl cellulose (HPMC) | 0.1 | 10.0 |
| USP | Water for Injection (WFI) | 85.9 | 8590.0 |
| Total weight | | 100.0 | 100.0 |

The hydrogel was produced in a class 100000 clean room using depyrogenized, sterile utensils. A double lumen mixer with a two-wing impeller was precooled to a temperature of 8 ± 2 °C. 8590.0 g of WFI were then transferred into the mixer. The mixer impeller was turned on and 50.0 g of PEG-400 were and added to the mixer. 10.0 g of HPMC and 1350.0 g Pluronic F127 were mixed thoroughly in a separate container and then gradually added to the mixer. The gel was stirred for 1 hr until homogeneous, transparent, colorless solution was obtained.

Aseptic filtration and water evaporation was performed in a Class 100 environment. The mixer outlet was connected to a sterile silicone tube connected to a peristaltic pump and to a 0.2 micron filter. The gel was filtered to a double lumen evaporation flask.

The evaporation flaskwas connected to a cold trap and vacuum pump. Water evaporation was performed at a gel temperature of 19 - 30 °C. The evaporation process was stopped once the formulation water content reached 71.8%. The product was cooled to 8 ± 2 °C and filled in vials. Water content of the gel was determined by means of LOD (loss on drying) and determination of water concentration.

### EXAMPLE 4

A non-limiting embodiment of the method that comprises a step of adding a pharmaceutical agent is herein described. 5 kg of reverse thermal gelation hydrogel were prepared from the components listed in Table 4.

**Table 4**

| Grade | Raw material | % (w/w) | g/batch (w/w) |
|---|---|---|---|
| USP | Pluronic F127 or | 27.0 | 1350.0 |
| NF | Poloxamer 407 NF | | |
| USP | Polyethylene glycol 400 (PEG-400) | 0.9 | 45.0 |
| USP | Lidocaine HCl | 0.1 | 5.0 |
| USP | Hydroxypropyl methyl cellulose (HPMC) | 0.2 | 10.0 |
| | WFI)(Water for Injection | 71.8 | 3590.0 |
| | Total weight | 100.0 | 5000.0 |

The hydrogel was produced in a similar fashion to that disclosed in Example 1 above. Production of the hydrogel and aseptic filtration were performed at a temperature of 8 ± 2 °C (i.e. near Tₘᵢₙ).

The hydrogel production was performed in a class 100000 clean room using a double lumen mixer with a two-wing impeller. The mixer was precooled to 8 ± 2 °C. 3590.0 g of WFI were transferred into the mixer. The mixer impeller was then turned on. 5.0 g of lidocaine HCl powder and 45.0 g PEG-400 were added to the mixer. 10.0 g of HPMC and 130.0 g of Pluronic F127 were thoroughly mixed in a separate container and then gradually added to the mixer. The gel was stirred for 2 h until a homogeneous, transparent, colorless solution was obtained.

Aseptic filtration was performed in a Class 100 environment. The mixer outlet was connected to a short sterile silicone tube connected to a peristaltic pump and to a double lumen stainless steel tube connected to a 0.2 micron filter. Both the double lumen stainless steel tube and the filter were maintained at a temperature of 8 ± 2 °C. The gel was filtered into a double lumen vessel maintained at a temperature of 8 ± 2 °C. The viscosity of the gel following filtration was found to be equal to that before filtration. The filtered gel, comprising a therapeutic composition comprising 0.1% lidocaine, was then transferred to vials. This therapeutic formulation can be applied topically to the internal and external surfaces of organs of the body.

The examples above are non-limiting and are presented only as particular cases of procedures based on the utilization of the above and similar components. Different therapeutic agents can be utilized according to the therapeutic application needs. Different compositions can be prepared that confer to the final admixture specific adherence properties to different body organ tissues according to the characteristics of said tissues. For example, a mucosal tissue may require a higher HPMC concentration to allow the composition to better adhere to its surface while a very elastic, mobile tissue may require an added amount of PEG-400 to add to the composition's elasticity.

## Claims

1. A method for production of a sterile thermoreversible hydrogel, said hydrogel having a temperature Tₘᵢₙ lower than its gelation temperature, at which the viscosity reaches an at least local minimum, **characterized in that** said process comprises:
mixing dry components of said hydrogel in a separate container;
dissolving in water, within a predetermined temperature range relative to said Tₘᵢₙ, components of said sterile thermoreversible hydrogel;
mixing said components until a thermoreversible gel is obtained; and,
filtering said thermoreversible gel within a predetermined temperature of said Tₘᵢₙ; wherein said components comprise between 18% and 40% (w/w) ethylene oxide/propylene oxide block copolymer, between 0.05% and 2% (w/w) HPMC or CMC, between 0.1% and 10% (w/w) PEG-400, and the balance water; and
wherein said predetermined temperature range is ± 4 °C of said Tₘᵢₙ.

2. A method for production of a sterile thermoreversible hydrogel, said hydrogel having a temperature Tₘᵢₙ at which the viscosity reaches an at least local minimum, **characterized in that** said process comprises:
mixing dry components of said hydrogel in a separate container;
dissolving components of said sterile thermoreversible hydrogel in a quantity of water sufficiently large to prevent formation of a gel with thermal gelation characteristics;
filtering said gel within a predetermined temperature of said Tₘᵢₙ, wherein said predetermined temperature range is ± 4 °C of said Tₘᵢₙ; and,
concentrating said gel by evaporating at least part of said water under vacuum, thereby forming a thermoreversible gel; wherein said components comprise between 18% and 40% (w/w) ethylene oxide/propylene oxide block copolymer, between 0.05% and 2% (w/w) HPMC or CMC, between 0.1% and 10% (w/w) PEG- 400, and the balance water.

3. The method according to either one of claims 1 or 2, wherein said components comprise between 20% and 30% (w/w) ethylene oxide/propylene oxide block copolymer, between 0.05% and 0.5% (w/w) hydroxypropylmethylcellulose (HPMC), between 0.1% and 2.5% (w/w) PEG-400, and the balance water.

4. The method according to any one of claims 1, 2, or 3, further including a step of adding an effective amount of a therapeutic agent.

5. The method according to any one of claims 1 to 4, **characterized in that** said components additionally comprise at least one component chosen from the group consisting of adhesive and thickening compounds; bonding agents; pH-modifying substances; diffusion coatings; plasticizers; water soluble polymers; water-soluble substances chosen from the group consisting of urea, salts, sugars, sugar alcohols, and any combination thereof; swellable excipients; matrix forming polymers; tight junction modifiers; permeability enhancers; surfactants; charged polymers; dimethyl sulfoxide; decylmethyl sulfoxide; tert-butylcyclohexanol; fatty acids; fatty acid esters; fatty acid salts; ethanol; nicotinamide; perfluoropolyethers; monoterpene ketones; and tris(hydroxymethyl)aminomethane.

6. The method according to claim 5, **characterized in that** said bonding agent is selected from the group consisting of polycarbophil, cellulose, microcrystalline cellulose, HPMC, hydroxypropylcellulose (HPC), low-substituted hydroxypropylcellulose (L-HPC) , dicalcium phosphate, lactose, sucrose ethylcellulose, hydroxypropymethylcellulose acetate succinate (HPMCAS), PVP, vinylpyrrolidone/vinyl acetate copolymer, polyethylene glycol, polyethylene oxide, polymethacrylates, polyvinyl alcohols (PVA), partially hydrolysed polyvinyl acetate (PVAc), polysaccharides, fats and fatty acids and any combination thereof.

7. The method according to claim 5, **characterized in that** said pH-modifying substance is chosen from the group consisting of adipic acid, malic acid, L-arginine, ascorbic acid, aspartic acid, benzenesulfonic acid, benzoic acid, succinic acid, citric acid, ethanesulphonic acid, 2-hydroxyethanesulphonic acid, fumaric acid, gluconic acid, glucuronic acid, glutamic acid, potassium hydrogen tartrate, maleic acid, malonic acid, methanesulfonic acid, toluenesulfonic acid, trometamol, tartaric acid, hydrochloric acid, sodium hydroxide, phosphate salts, tris and any combination thereof.

8. The method according to claim 5, **characterized in that** said diffusion coating is selected from the group consisting of ethylcelluloses, polymethacrylates, cellulose acetate, cellulose acetate butyrate or any combination thereof.

9. The method according to claim 5, **characterized in that** said plasticizer is selected from the group consisting of triethyl citrate, tributyl citrate, or acetyl triethyl citrate, dimethyl phthalate, diethyl phthalate, and dibutyl phthalate, benzoic acid, benzoic esters, other aromatic carboxylic esters, trimellithic esters, aliphatic dicarboxylic esters, dialkyl adipates, sebacic esters, tartaric esters, glycerol monoacetate, glycerol diacetate, glycerol triacetate, polyols, fatty acids, glycerol monostearates, acetylated fatty acid glycerides, natural oils, miglyol, fatty acid alcohols, cetyl alcohol, cetylstearyl alcohol, and any combination thereof.

10. The method according to claim 5, **characterized in that** said water-soluble substance comprises at least one substance chosen from the group consisting of:
salts chosen from the group consisting of sodium chloride, potassium chloride, and ammonium chloride;
at least one sugar chosen from the group consisting of sucrose, lactose, glucose, fructose, and maltose;
and, at least one sugar alcohol chosen from the group consisting of mannitol, sorbitol, xylitol, and lactitol.

11. The method according to claim 5, **characterized in that** said water-soluble polymer is selected from the group consisting of polyethylene glycols, PVP, PVA, HPC, hydroxyethylcelluloses (HEC), MC, dextrins, maltodextrins, cylcodextrins, dextrans, and any combination thereof.

12. The method according to claim 5, **characterized in that** said swellable excipient is chosen from the group consisting of polyvinylpyrrolidones, crospovidones, crosslinked sodium carboxymethylcellulose, crosslinked sodium carboxymethylstarch, polyethylene oxides, polymethyacrylates, low-substituted hydroxypropylmethylcellulose (L-HPC), cellulose acetate, ethylcellulose and polymethacrylates, high-molecular weight polyethylene oxides, xanthan gum, copolymers of vinylpyrrolidone and vinyl acetate, polyvinylpyrrolidones, crospovidones, poly(hydroxyalkyl methacrylate), alginates, galactomannans, and any combination thereof.

13. The method according to claim 5, **characterized in that** said matrix forming polymer is selected from the group consisting of hydroxyethylmethylcelluloses, hydroxypropylcelluloses, hydroxyethylcelluloses, methylcelluloses, ethylcelluloses, alkylcelluloses, hydroxyalkyl-celluloses, hydroxyalkylmethylcelluloses, sodium carboxymethylcelluloses, alginates, galactomannans, xanthans, polyethylene oxides, polyacrylic acids, polymethacrylic acids, polyvinyl alcohols, partially hydrolysed polyvinyl acetate, polyvinylpyrrolidone, agar, pectin, gum arabic, tragacanth, gelatin, starches, poly(propylene oxide) (PPO), poly(lactide-co-glycolic acid) (PLGA), poly(N-isopropylacrylamide) (PNIPAM), poly(propylene fumarate) (PPF), polyurethane (PU), poly(organophosphazene) (POP), stearic acid, poly(acrylic acid), glyceryl stearate, cetearyl alcohol, sodium stearoyl lactylate, hydroxy-lanolin, and any combination thereof.

14. The method according to claim 5, **characterized in that** at least one of the following is true:
said surfactant is chosen from the group consisting of polysorbates, sodium dodecyl sulfate, and dextran sulfate;
said charged polymer is chosen from the group consisting of chitosan, polyarginine, polylysine, and alginate; and,
said monoterpene ketone is chosen from the group consisting of (-)menthol, (-) menthone, peppermint oil, and spearmint oil.

## Patentansprüche

1. Verfahren zur Herstellung eines sterilen thermoreversiblen Hydrogels, wobei das Hydrogel eine Temperatur Tₘᵢₙ hat, die niedriger als seine Gelierungstemperatur ist, bei der die Viskosität ein mindestens lokales Minimum erreicht, **dadurch gekennzeichnet, dass** das Verfahren umfasst:
Mischen trockener Komponenten des Hydrogels in einem separaten Behälter;
Auflösen der Komponenten des sterilen thermoreversiblen Hydrogels in Wasser innerhalb eines vorbestimmten Temperaturbereichs relativ zu der Tₘᵢₙ;
Mischen der Komponenten, bis ein thermoreversibles Gel erhalten wird; und
Filtern des thermoreversiblen Gels innerhalb einer vorbestimmten Temperatur der Tₘᵢₙ; wobei die Komponenten zu zwischen 18 und 40 Gew.-% Ethylenoxid/Propylenoxid-Blockcopolymer, zu zwischen 0,05 und 2 Gew.-% HPMC oder CMC, zu zwischen 0,1 und 10 Gew.-% PEG-400 und den Rest Wasser umfassen; und
wobei der vorbestimmte Temperaturbereich ± 4 °C der Tₘᵢₙ ist.

2. Verfahren zur Herstellung eines sterilen thermoreversiblen Hydrogels, wobei das Hydrogel eine Temperatur Tₘᵢₙ hat, bei der die Viskosität ein mindestens lokales Minimum erreicht, **dadurch gekennzeichnet, dass** das Verfahren umfasst:
Mischen trockener Komponenten des Hydrogels in einem separaten Behälter;
Auflösen von Komponenten des sterilen thermoreversiblen Hydrogels in einer Wassermenge, die groß genug ist, um die Bildung eines Gels mit thermischen Geliereigenschaften zu verhindern;
Filtern des Gels innerhalb einer vorbestimmten Temperatur der Tₘᵢₙ, wobei der vorbestimmte Temperaturbereich ± 4 °C der Tₘᵢₙ beträgt; und,
Konzentrieren des Gels durch Verdampfen mindestens eines Teils des Wassers unter Vakuum, wodurch ein thermoreversibles Gel gebildet wird; wobei die Komponenten zu zwischen 18 und 40 Gew.-% Ethylenoxid/Propylenoxid-Blockcopolymer, zu zwischen 0,05 und 2 Gew.-% HPMC oder CMC, zu zwischen 0,1 und 10 Gew.-% PEG-400 und den Rest Wasser umfassen.

3. Verfahren nach einem der Ansprüche 1 oder 2, wobei die Komponenten zu zwischen 20 und 30 Gew.-% Ethylenoxid/Propylenoxid-Blockcopolymer, zu zwischen 0,05 und 0,5 Gew.-% Hydroxypropylmethylcellulose (HPMC), zu zwischen 0,1 und 2,5 Gew.-% PEG-400 und den Rest Wasser umfassen.

4. Verfahren nach einem der Ansprüche 1, 2 oder 3, ferner umfassend einen Schritt des Hinzufügens einer wirksamen Menge eines therapeutischen Mittels.

5. Verfahren nach einem der Ansprüche 1 bis 4, **dadurch gekennzeichnet, dass** die Komponenten zusätzlich mindestens eine Komponente umfassen, die ausgewählt ist aus der Gruppe bestehend aus klebenden und verdickenden Verbindungen; Bindemitteln; pHmodifizierenden Substanzen; Diffusionsbeschichtungen; Weichmachern; wasserlöslichen Polymeren; wasserlöslichen Substanzen, ausgewählt aus der Gruppe bestehend aus Harnstoff, Salzen, Zuckern, Zuckeralkoholen und Kombinationen davon; quellbaren Hilfsstoffen; matrixbildenden Polymeren; Tight-Junction-Modifikatoren; Permeabilitätsverstärkern; Tensiden; geladenen Polymeren; Dimethylsulfoxid; Decylmethylsulfoxid; tert-Butylcyclohexanol; Fettsäuren; Fettsäureestern; Fettsäuresalzen; Ethanol; Nikotinamid; Perfluoropolyethern; Monoterpenketonen; und Tris(hydroxymethyl)aminomethan.

6. Verfahren nach Anspruch 5, **dadurch gekennzeichnet, dass** das Bindemittel ausgewählt ist aus der Gruppe bestehend aus Polycarbophil, Cellulose, mikrokristalliner Cellulose, HPMC, Hydroxypropylcellulose (HPC), niedrig substituierter Hydroxypropylcellulose (L-HPC), Dicalciumphosphat, Lactose, Saccharose-Ethylcellulose, Hydroxypropymethylcelluloseacetatsuccinat (HPMCAS), PVP, Vinylpyrrolidon/Vinylacetat-Copolymer, Polyethylenglykol, Polyethylenoxid, Polymethacrylaten, Polyvinylalkoholen (PVA), teilhydrolysiertem Polyvinylacetat (PVAc), Polysacchariden, Fetten und Fettsäuren und Kombinationen davon.

7. Verfahren nach Anspruch 5, **dadurch gekennzeichnet, dass** die pH-modifizierende Substanz ausgewählt ist aus der Gruppe bestehend aus Adipinsäure, Äpfelsäure, L-Arginin, Ascorbinsäure, Asparaginsäure, Benzolsulfonsäure, Benzoesäure, Bernsteinsäure, Zitronensäure, Ethansulfonsäure, 2-Hydroxyethansulfonsäure, Fumarsäure, Gluconsäure, Glucuronsäure, Glutaminsäure, Kaliumhydrogentartrat, Maleinsäure, Malonsäure, Methansulfonsäure, Toluolsulfonsäure, Trometamol, Weinsäure, Salzsäure, Natriumhydroxid, Phosphatsalzen, Tris und Kombinationen davon.

8. Verfahren nach Anspruch 5, **dadurch gekennzeichnet, dass** die Diffusionsbeschichtung ausgewählt ist aus der Gruppe bestehend aus Ethylcellulosen, Polymethacrylaten, Celluloseacetat, Celluloseacetatbutyrat oder Kombinationen davon.

9. Verfahren nach Anspruch 5, **dadurch gekennzeichnet, dass** der Weichmacher ausgewählt ist aus der Gruppe bestehend aus Triethylcitrat, Tributylcitrat oder Acetyltriethylcitrat, Dimethylphthalat, Diethylphthalat und Dibutylphthalat, Benzoesäure, Benzoesäureestern, anderen aromatischen Carbonsäureestern, Trimellithsäureestern, aliphatischen Dicarbonsäureestern, Dialkyladipaten, Sebacinsäureestern, Weinsäureestern, Glycerinmonoacetat, Glycerindiacetat, Glycerintriacetat, Polyolen, Fettsäuren, Glycerinmonostearaten, acetylierten Fettsäureglyceriden, natürlichen Ölen, Miglyol, Fettsäurealkoholen, Cetylalkohol, Cetylstearylalkohol und Kombinationen davon.

10. Verfahren nach Anspruch 5, **dadurch gekennzeichnet, dass** die wasserlösliche Substanz mindestens eine Substanz umfasst, die ausgewählt ist aus der Gruppe bestehend aus:
Salzen, die ausgewählt sind aus der Gruppe bestehend aus Natriumchlorid, Kaliumchlorid und Ammoniumchlorid;
mindestens einem Zucker, der ausgewählt ist aus der Gruppe bestehend aus Saccharose, Laktose, Glukose, Fruktose und Maltose;
und mindestens einem Zuckeralkohol, der ausgewählt ist aus der Gruppe bestehend aus Mannit, Sorbit, Xylit und Lactit.

11. Verfahren nach Anspruch 5, **dadurch gekennzeichnet, dass** das wasserlösliche Polymer ausgewählt ist aus der Gruppe bestehend aus Polyethylenglykolen, PVP, PVA, HPC, Hydroxyethylcellulosen (HEC), MC, Dextrinen, Maltodextrinen, Cylcodextrinen, Dextranen und Kombinationen davon.

12. Verfahren nach Anspruch 5, **dadurch gekennzeichnet, dass** der quellbare Hilfsstoff ausgewählt ist aus der Gruppe bestehend aus Polyvinylpyrrolidonen, Crospovidonen, vernetzter Natriumcarboxymethylcellulose, vernetzter Natriumcarboxymethylstärke, Polyethylenoxiden, Polymethyacrylaten, niedrig substituierter Hydroxypropylmethylcellulose (L-HPC), Celluloseacetat, Ethylcellulose und Polymethacrylaten, hochmolekularen Polyethylenoxiden, Xanthangummi, Copolymeren von Vinylpyrrolidon und Vinylacetat, Polyvinylpyrrolidonen, Crospovidonen, Poly(hydroxyalkylmethacrylat), Alginaten, Galactomannanen und Kombinationen davon.

13. Verfahren nach Anspruch 5, **dadurch gekennzeichnet, dass** das matrixbildende Polymer ausgewählt ist aus der Gruppe bestehend aus Hydroxyethylmethylcellulosen, Hydroxypropylcellulosen, Hydroxyethylcellulosen, Methylcellulosen, Ethylcellulosen, Alkylcellulosen, Hydroxyalkylcellulosen, Hydroxyalkylmethylcellulosen, Natriumcarboxymethylcellulosen, Alginaten, Galactomannanen, Xanthanen, Polyethylenoxiden, Polyacrylsäuren, Polymethacrylsäuren, Polyvinylalkoholen, teilhydrolysiertem Polyvinylacetat, Polyvinylpyrrolidon, Agar, Pektin, Gummi arabicum, Tragant, Gelatine, Stärken, Poly(propylenoxid) (PPO), Poly(lactid-co-glycolsäure) (PLGA), Poly(N-isopropylacrylamid) (PNIPAM), Poly(propylenfumarat) (PPF), Polyurethan (PU), Poly(organophosphazen) (POP), Stearinsäure, Poly(acrylsäure), Glycerinstearat, Cetearylalkohol, Natriumstearoyllactylat, Hydroxy-Lanolin und Kombinationen davon.

14. Verfahren nach Anspruch 5, **dadurch gekennzeichnet, dass** mindestens eine der folgenden Aussagen zutrifft:
das Tensid ist ausgewählt aus der Gruppe bestehend aus Polysorbaten, Natriumdodecylsulfat und Dextransulfat;
das geladene Polymer ist ausgewählt aus der Gruppe bestehend aus Chitosan, Polyarginin, Polylysin und Alginat; und
das Monoterpenketon ist ausgewählt aus der Gruppe bestehend aus (-)Menthol, (-)Menthon, Pfefferminzöl und Spearmintöl.

## Revendications

1. Procédé pour la production d'un hydrogel thermoréversible stérile, ledit hydrogel ayant une température Tₘᵢₙ inférieure à sa température de gélification, à laquelle la viscosité atteint un minimum au moins local, **caractérisé en ce que** ledit processus comprend :
le mélange de composants secs dudit hydrogel dans un récipient séparé ;
la dissolution dans de l'eau, dans une plage de température prédéterminée par rapport à ladite Tₘᵢₙ, de composants dudit hydrogel thermoréversible stérile ;
le mélange desdits composants jusqu'à ce qu'un gel thermoréversible soit obtenu ; et,
le filtrage dudit gel thermoréversible à une température prédéterminée de ladite Tₘᵢₙ ; dans lequel lesdits composants comprennent entre 18 % et 40 % (en poids) de copolymère séquencé d'oxyde d'éthylène/oxyde de propylène, entre 0,05 % et 2 % (en poids) de HPMC ou de CMC, entre 0,1 % et 10 % (en poids) de PEG-400, et le solde d'eau ; et
dans lequel ladite plage de température prédéterminée est à ± 4 °C de ladite Tₘᵢₙ.

2. Procédé pour la production d'un hydrogel thermoréversible stérile, ledit hydrogel ayant une température Tₘᵢₙ à laquelle la viscosité atteint un minimum au moins local, **caractérisé en ce que** ledit processus comprend :
le mélange de composants secs dudit hydrogel dans un récipient séparé ;
la dissolution de composants dudit hydrogel thermoréversible stérile dans une quantité d'eau suffisamment grande pour empêcher la formation d'un gel avec des caractéristiques de gélification thermique ;
le filtrage dudit gel à plus ou moins une température prédéterminée de ladite Tₘᵢₙ, dans lequel ladite plage de température prédéterminée est à ± 4 °C de ladite Tₘᵢₙ ; et,
la concentration dudit gel en évaporant au moins une partie de ladite eau sous vide, ce qui forme un gel thermoréversible ; dans lequel lesdits composants comprennent entre 18 % et 40 % (en poids) de copolymère séquencé d'oxyde d'éthylène/oxyde de propylène, entre 0,05 % et 2 % (en poids) de HPMC ou de CMC, entre 0,1 % et 10 % (en poids) de PEG- 400, et le solde d'eau.

3. Procédé selon l'une quelconque des revendications 1 ou 2, dans lequel lesdits composants comprennent entre 20 % et 30 % (en poids) de copolymère séquencé d'oxyde d'éthylène/oxyde de propylène, entre 0,05 % et 0,5 % (en poids) d'hydroxypropylméthylcellulose (HPMC), entre 0,1 % et 2,5 % (en poids) de PEG-400, et le solde d'eau.

4. Procédé selon l'une quelconque des revendications 1, 2, ou 3, incluant en outre une étape d'ajout d'une quantité efficace d'un agent thérapeutique.

5. Procédé selon l'une quelconque des revendications 1 à 4, **caractérisé en ce que** lesdits composants comprennent en outre au moins un composant choisi dans le groupe constitué de composés adhésifs et épaississants ; agents de liaison ; substances modifiant le pH ; revêtements de diffusion ; plastifiants ; polymères hydrosolubles ; substances hydrosolubles choisies dans le groupe constitué d'urée, sels, sucres, alcools de sucre, et n'importe quelle combinaison de ceux-ci ; excipients pouvant gonfler ; polymères de formation de matrice ; modificateurs de jonction étanche ; agents renforçant la perméabilité ; agents tensioactifs ; polymères chargés ; sulfoxyde de diméthyle ; sulfoxyde de décylméthyle ; tert-butylcyclohexanol ; acides gras ; esters d'acide gras ; sels d'acide gras ; éthanol ; nicotinamide ; perfluoropolyéthers ; cétones monoterpéniques ; et tris(hydroxyméthyl)aminométhane.

6. Procédé selon la revendication 5, **caractérisé en ce que** ledit agent de liaison est choisi dans le groupe constitué de polycarbophile, cellulose, cellulose microcristalline, HPMC, hydroxypropylcellulose (HPC), hydroxypropylcellulose faiblement substituée (L-HPC), phosphate dicalcique, lactose, saccharose éthylcellulose, acétate-succinate d'hydroxypropyméthylcellulose (HPMCAS), PVP, copolymère de vinylpyrrolidone/acétate de vinyle, polyéthylène glycol, oxyde de polyéthylène, polyméthacrylates, alcools polyvinyliques (PVA), acétate de polyvinyle (PVAc) partiellement hydrolysé, polysaccharides, matières grasses et acides gras et n'importe quelle combinaison de ceux-ci.

7. Procédé selon la revendication 5, **caractérisé en ce que** ladite substance modifiant le pH est choisie dans le groupe constitué d'acide adipique, acide malique, L-arginine, acide ascorbique, acide aspartique, acide benzènesulfonique, acide benzoïque, acide succinique, acide citrique, acide éthanesulfonique, acide 2-hydroxyéthanesulfonique, acide fumarique, acide gluconique, acide glucuronique, acide glutamique, hydrogénotartrate de potassium, acide maléique, acide malonique, acide méthanesulfonique, acide toluènesulfonique, trométamol, acide tartrique, acide chlorhydrique, hydroxyde de sodium, sels phosphate, tris et n'importe quelle combinaison de ceux-ci.

8. Procédé selon la revendication 5, **caractérisé en ce que** ledit revêtement de diffusion est choisi dans le groupe constitué d'éthylcelluloses, polyméthacrylates, acétate de cellulose, acétobutyrate de cellulose ou n'importe quelle combinaison de ceux-ci.

9. Procédé selon la revendication 5, **caractérisé en ce que** ledit plastifiant est choisi dans le groupe constitué de citrate de triéthyle, citrate de tributyle, ou citrate d'acétyl-triéthyle, phtalate de diméthyle, phtalate de diéthyle, et phtalate de dibutyle, acide benzoïque, esters benzoïques, autres esters carboxyliques aromatiques, esters trimellitiques, esters dicarboxyliques aliphatiques, adipates de dialkyle, esters sébaciques, esters tartriques, monoacétate de glycérol, diacétate de glycérol, triacétate de glycérol, polyols, acides gras, monostéarates de glycérol, glycérides d'acides gras acétylés, huiles naturelles, miglyol, alcools d'acide gras, alcool cétylique, alcool cétylstéarylique, et n'importe quelle combinaison de ceux-ci.

10. Procédé selon la revendication 5, **caractérisé en ce que** ladite substance hydrosoluble comprend au moins une substance choisie dans le groupe constitué de :
sels choisis dans le groupe constitué de chlorure de sodium, chlorure de potassium, et chlorure d'ammonium ;
au moins un sucre choisi dans le groupe constitué de saccharose, lactose, glucose, fructose, et maltose ;
et, au moins un alcool de sucre choisi dans le groupe constitué de mannitol, sorbitol, xylitol, et lactitol.

11. Procédé selon la revendication 5, **caractérisé en ce que** ledit polymère hydrosoluble est choisi dans le groupe constitué de polyéthylène glycols, PVP, PVA, HPC, hydroxyéthylcelluloses (HEC), MC, dextrines, maltodextrines, cyclodextrines, dextranes, et n'importe quelle combinaison de ceux-ci.

12. Procédé selon la revendication 5, **caractérisé en ce que** ledit excipient pouvant gonfler est choisi dans le groupe constitué de polyvinylpyrrolidones, crospovidones, carboxyméthylcellulose sodique réticulée, carboxyméthylamidon sodique réticulé, oxydes de polyéthylène, polyméthylacrylates, hydroxypropylméthylcellulose faiblement substituée (L-HPC), acétate de cellulose, éthylcellulose et polyméthacrylates, oxydes de polyéthylène à masse moléculaire élevée, gomme de xanthane, copolymères de vinylpyrrolidone et d'acétate de vinyle, polyvinylpyrrolidones, crospovidones, poly(méthacrylate d'hydroxyalkyle), alginates, galactomannanes, et n'importe quelle combinaison de ceux-ci.

13. Procédé selon la revendication 5, **caractérisé en ce que** ledit polymère de formation de matrice est choisi dans le groupe constitué d'hydroxyéthylméthylcelluloses, hydroxypropylcelluloses, hydroxyéthylcelluloses, méthylcelluloses, éthylcelluloses, alkylcelluloses, hydroxyalkyl-celluloses, hydroxyalkylméthylcelluloses, carboxyméthylcelluloses sodiques, alginates, galactomannanes, xanthanes, oxydes de polyéthylène, acides polyacryliques, acides polyméthacryliques, alcools polyvinyliques, acétate de polyvinyle partiellement hydrolysé, polyvinylpyrrolidone, agar-agar, pectine, gomme arabique, tragacanthe, gélatine, amidons, poly(oxyde de propylène) (PPO), poly(lactide-co-acide glycolique) (PLGA), poly(N-isopropylacrylamide) (PNIPAM), poly(fumarate de propylène) (PPF), polyuréthane (PU), poly(organophosphazène) (POP), acide stéarique, acide poly(acrylique), stéarate de glycéryle, alcool cétéarylique, stéaroyl-lactylate sodique, hydroxy-lanoline, et n'importe quelle combinaison de ceux-ci.

14. Procédé selon la revendication 5, **caractérisé en ce qu'**au moins un de ce qui suit est vrai :
ledit agent tensioactif est choisi dans le groupe constitué de polysorbates, dodécylsulfate de sodium, et sulfate de dextrane ;
ledit polymère chargé est choisi dans le groupe constitué de chitosane, polyarginine, polylysine, et alginate ; et,
ladite cétone monoterpénique est choisie dans le groupe constitué de (-)menthol, (-)menthone, essence de menthe poivrée, et essence de menthe verte.
